(19) Europäisches Patentamt
European Patent Office
Office européen des brevets

(11) **EP 4 556 931 A1**

(12) **EUROPEAN PATENT APPLICATION**

(43) Date of publication:
**21.05.2025 Bulletin 2025/21**

(21) Application number: **23210349.9**

(22) Date of filing: **16.11.2023**

(51) International Patent Classification (IPC):
**G01R 33/24** (2006.01)    **A61B 5/0536** (2021.01)
**A61B 5/055** (2006.01)    **G01R 33/48** (2006.01)

(52) Cooperative Patent Classification (CPC):
**G01R 33/4808; A61B 5/0536; A61B 5/055;**
**G01R 33/246;** G01R 33/5608

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **KATSCHER, Ulrich Wolfgang**
**Eindhoven (NL)**
• **FINDEKLEE, Christian**
**Eindhoven (NL)**

(74) Representative: **Philips Intellectual Property &**
**Standards**
**High Tech Campus 52**
**5656 AG Eindhoven (NL)**

(54) **SYSTEMS AND METHODS FOR ELECTRICAL PROPERTIES TOMOGRAPHY**

(57) Methods and systems for electric properties tomography imaging are presented. Maps of electrical properties such as conductivity and permittivity maps of an object are constructed with improved accuracy, in particular for objects having shapes with insufficient axial symmetry, thereby correcting for shortcoming caused by Transceive Phase Assumption in electric properties tomography.

Fig. 4A

EP 4 556 931 A1

## Description

### TECHNICAL FIELD

The subject matter described herein relates to electrical properties tomography, in particular to deriving electrical conductivity and permittivity maps based on measurements with a magnetic resonance system.

### BACKGROUND

Electrical properties tomography (EPT) is an imaging method that uses a magnetic resonance (MR) system to non-invasively derive the spatial distribution of the conductivity $\sigma$ and permittivity $\varepsilon$ of an imaged subject from measurement of magnitude and phase of the radiofrequency (RF) transmit field B1 (i.e., measurement of complex B1 map), followed by a subsequent postprocessing of the complex B1 map according to Maxwell's equations. An extensive review of EPT is to be found in Leijsen R., et al., "Electrical Properties Tomography: A Methodological Review", Diagnostics 2021, 11, 176, https://doi.org/10.3390/diagnostics11020176, which is hereby incorporated by reference in its entirety.

While the B1 magnitude can be measured exactly by well-established mapping techniques, the measured B1 phase is always a superposition of the B1 phase components belonging to RF transmission and RF reception, yielding the so-called B1 transceive phase. Standard practice is to divide the B1 transceive phase by two to obtain an estimation of the required B1 transmit field (so-called "Transceive Phase Assumption", TPA).

The information included in this Background section of the specification, including any references cited herein and any description or discussion thereof, is included for technical reference purposes only and is not to be regarded as subject matter by which the scope of the disclosure is to be bound.

### SUMMARY

The invention proposes to address the shortcoming of the validity of the TPA, which is progressively violated with increasing main static magnetic field B0, e.g., typically above 1.5 Tesla (T), and/or increasing object size, e.g., typically larger head size, unless the object shape shows sufficient axial symmetry. This limitation causes artefacts in the maps of electrical properties. Accordingly, the objective of the invention is to reduce the artefacts considerably, improving the accuracy of the electrical properties tomography results.

In an aspect of the invention a system is provided for electrical properties tomography imaging, comprising: a memory storing machine executable instructions; a computational system, wherein execution of the machine executable instructions causes the computational system to:

- receive a first magnetic resonance imaging data in a first position of an object and ascertain a first set of B1 magnitudes and a first set of B 1 phases from the first magnetic resonance imaging data;
- receive a second magnetic resonance imaging data in a second position of the object and ascertain a second set of B1 magnitudes and a second set of B1 phases from the second magnetic resonance imaging data;
- determine a resultant map of electrical property by:

    constructing a first map of electrical property based on the first set of B1 magnitudes and the first set of B 1 phases;
    constructing a second map of electrical property based on the second set of B1 magnitudes and the second set of B 1 phases, registering the first map of electrical property to the second map of electrical property and averaging the registered maps of electrical property; or
    mapping the first set of B1 magnitudes and B 1 phases to the second set of B 1 magnitudes and B 1 phases and averaging the mapped sets of B1 magnitudes and B 1 phases;

- output the resultant map of electrical property to a display screen.

Registering the first map of electrical property with the second map of electrical property may comprise registering based on anatomy, on anatomic marks or anatomical regions. Mapping the first set of B1 magnitudes and B 1 phases to the second set of B1 magnitudes and B 1 phases may comprise mapping based on anatomic marks.

In an embodiment of the system the second position is at about 180 degrees yaw from the first position. For a medical system this means that a patient has been introduced with e.g. head first (HF) in the scanner for obtaining the first magnetic resonance imaging data of a portion of the body, then the support on which the patient has been positioned is turned about 180 degrees, so that the second magnetic resonance data is obtained by scanning the same portion of the body of the patient as in the first position, but now in the configuration wherein the patient has been introduced with e.g. feet first (FF) in the bore of the scanner. In an embodiment, the support is configured to allow orientation of the object from the first position to the second position, for example by a rotatable support that allows at least a 180 degrees yaw. A rotatable support, e.g., patient table, facilitates workflow avoiding movements of the patients themselves. The person skilled in the art should understand that either one of HF and FF can be associated with the first magnetic resonance imaging data, while the other one of HF and FF is associated with the second magnetic resonance imaging data.

In an embodiment of the system, two magnetic resonance imaging scanners are placed such that the direction of the main static magnetic fields of the two scanners are opposite to each other; and a support of the object is configured to allow translation from the first position in the first scanner to the second position in the second scanner. Potential benefit of this embodiment is avoidance of left-right asymmetries that can occur especially at higher magnetic field strengths (i.e. 3 T and above for body imaging, 7 T and above for head imaging). The bore of the scanner is associated with a main static magnetic field in about horizontal position, e.g., superconducting cylindrical type magnet.

In an alternative embodiment, for example an open MRI scanner is utilized wherein the main static magnetic field B0 is oriented vertically. The first position in this configuration is either one of supine and prone position of the patient which is thus associated with the first magnetic resonance imaging data, while the other one of supine and prone position of the patient is associated with the second magnetic resonance imaging data. The supine and prone positions are separated from each other by 180 degrees roll around the longitudinal axis of the patient.

In an alternative aspect of the invention, a system is provided for electrical properties tomography imaging, comprising: a memory storing machine executable instructions; a computational system, wherein execution of the machine executable instructions causes the computational system to:

- receive a magnetic resonance imaging data of an object and ascertain a first set of B1 magnitudes and a first set of B1 phases from the magnetic resonance imaging data;
- mirror the first set of B1 magnitudes to obtain a second set of B1 magnitudes;
- determine a resultant map of electrical property by:

   constructing a first map of electrical property based on the first set of B1 magnitudes and the first set of B1 phases, constructing a second map of electrical property based on the second set of B1 magnitudes and first set of B1 phases, averaging the first map of electrical property and the second map of electrical property; or averaging the first set and second sets of B1 magnitudes and by using the first set of B1 phases;

- output the resultant map of electrical property to a display screen.

This embodiment reduces the scan time and requires no repositioning of the object, e.g., releases the constraint on repositioning of the patient for the second scan.

In any of the embodiments of the system the mirroring can be performed line by line with individual pivot points.

In any of the embodiments of the system according to any of the aspects of the invention, the memory can be configured to store an image segmentation algorithm, wherein the image segmentation algorithm is configured for outputting one or more predetermined anatomical regions within magnetic resonance imaging data descriptive of a predetermined field of view of a subject; wherein execution of the machine executable instructions causes the computational system to provide an image segmentation comprising the one or more anatomical regions within the magnetic resonance imaging data in response to inputting the magnetic resonance imaging data into the image segmentation algorithm; wherein the resultant map of electrical property is output for the provided image segmentation.

In a further aspect a method of electrical properties tomography imaging is provided, comprising:

- receiving a first magnetic resonance imaging data in a first position of the object and ascertaining a first set of B1 magnitudes and a first set of B1 phases from the first magnetic resonance imaging data;
- receiving a second magnetic resonance imaging data in a second position of the object and ascertaining a second set of B1 magnitudes and a second set of B1 phases from the second magnetic resonance imaging data;
- determining a resultant map of electrical property by:

   constructing a first map of electrical property based on the first set of B1 magnitudes and the first set of B1 phases; constructing a second map of electrical property based on the second set of B1 magnitudes and the second set of B1 phases, registering the first map of electrical property to the second map of electrical property and averaging the registered maps of electrical property; or mapping the first set of B1 magnitudes and B1 phases to the second set of B1 magnitudes and B1 phases and averaging the mapped sets of B1 magnitudes and B1 phases;

- outputting the resultant map of electrical property to a display screen.

Some embodiments of this method may comprise: rotating the object on a rotatable support between the first position and the second position with a yaw of about 180 degrees; or providing the first and the second magnetic resonance data with two respective magnetic resonance imaging scanners placed such that the direction of the main static magnetic fields of the two scanners are opposite to each other, and translating the object on a support from the first position in the first scanner to the second position in the second scanner.

In yet a further aspect a method of electrical properties tomography imaging is provided, comprising:

- receiving a magnetic resonance imaging data of an object and ascertaining a first set of B1 magnitudes and first set of B1 phases from the magnetic resonance imaging data;
- mirroring the first set of B1 magnitudes to obtain a second set of B1 magnitudes;
- determining a resultant map of electrical property by:

  constructing a first map of electrical property based on the first set of B1 magnitudes and B 1 phases, constructing a second map of electrical property based on the second set of B 1 magnitudes and first set of B1 phases, averaging the first and second maps of electrical property; or
  averaging the first and second sets of B1 magnitudes, and using the first set of B1 phases;

- outputting the resultant map of electrical property to a display screen.

This method reduces the required scan time and releases the constraint on re-positioning of the patient for the second scan, which renders this method more practical for clinical routine.

Any of the embodiments of the methods may further comprise: providing an image segmentation comprising one or more anatomical regions within the magnetic resonance imaging data in response to inputting the magnetic resonance imaging data into an image segmentation algorithm; wherein outputting the resultant map of electrical property is for the provided image segmentation.

In any of the embodiments of the method the mirroring can be performed line by line with individual pivot points.

In any of the embodiments of the system or method, the electrical property may be either electrical conductivity, permittivity or both, electrical conductivity and permittivity. With other words, the resultant map of electrical property may be:

  an electrical conductivity map or data;
  a permittivity map or data;
  an electrical conductivity map and a permittivity map; or
  an electrical conductivity data and a permittivity data.

In yet a further aspect of the invention, a computer program is disclosed, comprising machine executable instructions, wherein execution of the machine executable instructions causes a computational system to perform all of the steps of the method according to any of the embodiments.

Features described above with respect to corresponding system embodiments apply also for method embodiments, with similar benefits.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the flow measurement system, as defined in the claims, is provided in the following written description of various embodiments of the disclosure and illustrated in the accompanying drawings.

**BRIEF DESCRIPTION OF THE DRAWINGS**

Illustrative embodiments of the present disclosure will be described with reference to the accompanying drawings, of which:

  Figure 1 illustrates an example of a medical system;
  Figure 2 illustrates a further example of a medical system;
  Figure 3 illustrates a flow chart of a method according to the invention;
  Figure 4A illustrates electrical conductivity simulation results for three different application examples using simplified geometrical models;
  Figure 4B illustrates electrical permittivity simulation results for three different application examples using simplified geometrical models;

Figure 5 illustrates a flow chart of an alternative method according to the invention;
Figure 6 illustrates electrical conductivity simulation results for simplified abdominal model according to the alternative method.

## DETAILED DESCRIPTION

For the purposes of promoting an understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

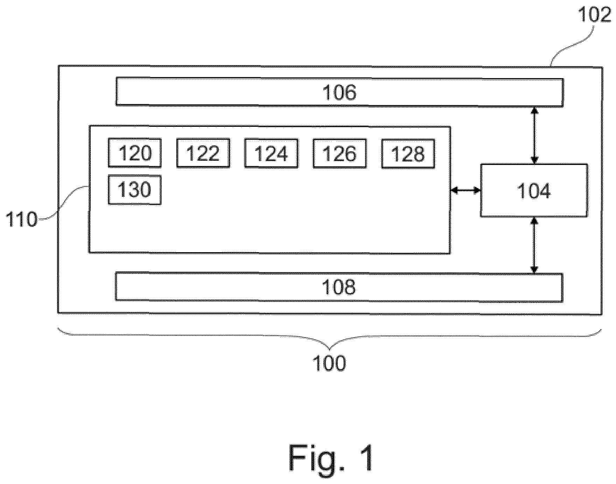

Fig. 1 illustrates an example of a system 100, which may be a medical system. In this example, the system comprises a computer 102. The computer 102 may represent one or more computer systems that are in one location or are distributed. The computer 102 is shown as containing a computational system 104. The computational system 104 may for example be one or more processing cores that are located at one or more locations. The computational system 104 is shown as being connected to an optional hardware interface 106. The hardware interface 106 may for example be used to connect the computational system 104 with other components of the medical system 100 if they are present. The hardware interface 106 may enable the computational system 104 to control such components.

The computer 102 is further shown as comprising an optional user interface 108. The user interface 108 may for example be used by an operator to control the operation and function of the medical system 100. The medical system 100 could be a standalone computer system but it could also be integrated into a magnetic resonance imaging system.

The computer 102 is further shown as comprising a memory 110. The memory 110 is intended to represent any combination of memory or storage device which is accessible to the computational system 104. The memory 110 may include volatile and non-volatile memory storage means and components.

The memory 110 is shown as containing machine-executable instructions 120. The machine-executable instructions 120 may be configured to enable the computational system 104 to perform basic data processing and image processing tasks, such as reconstructing magnetic resonance images. The machine-executable instructions 120 may also in some examples contain code which enables the computational system to control other components via the optional hardware interface 106.

The memory 110 may comprise image segmentation algorithm 122. The image segmentation algorithm 122 may be a standard magnetic resonance imaging image segmentation algorithm that is configured for outputting one or more predetermined anatomical regions for a magnetic resonance imaging data. This may be for a particular field of view or anatomical region of the subject, e.g., patient.

The memory 110 may comprise the measured magnetic resonance imaging data 124. The memory 110 may further comprise an image segmentation 126 that has been received from the image segmentation algorithm 122 by inputting the measured magnetic resonance imaging data 124. The memory 110 may comprise a selected image portion 128. This is one or more regions or anatomical regions that have been identified in the image segmentation 126. The selected image portion 128 may for example be selected using a predetermined criterion that is applied to the image segmentation 126. The memory may comprise one or a plurality of various algorithms 130 for deriving electrical properties based on magnetic resonance imaging data 124 and by optionally using any information present in the memory 110 and/or information that may be input by a user through the user interface 108.

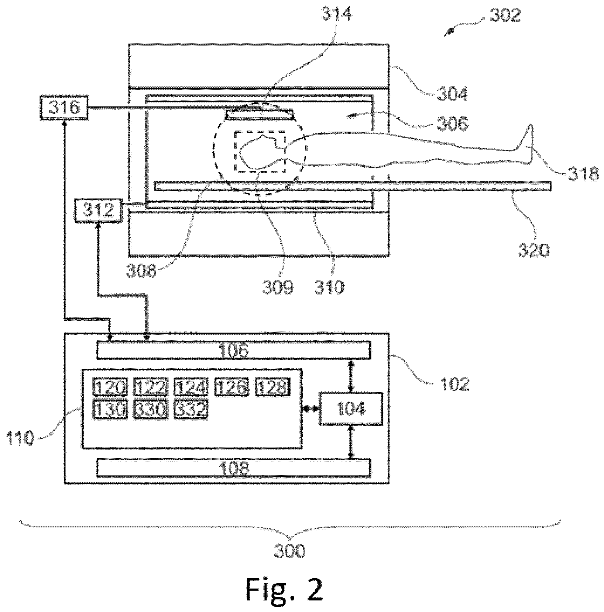

Fig. 2 illustrates a further example of a system 300, which may be a medical system. The medical system 300 comprises the medical system 100 in Fig. 1 and comprises a magnetic resonance imaging (MRI) system 302, or with other words a magnetic resonance imaging scanner, that is controlled by the computational system 104.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquired for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102.

The memory 110 is shown as containing pulse sequence commands 330. The pulse sequence commands are commands or data which can be converted into commands which can be used to control the magnetic resonance imaging system 302 to acquire k-space data 332. The memory 110 is further shown as comprising k-space data 332 that has been acquired by controlling the magnetic resonance imaging system 302 with the pulse sequence commands 330. The computational system 104 may also reconstruct the measured magnetic resonance imaging data 124 from the k-space data 332.

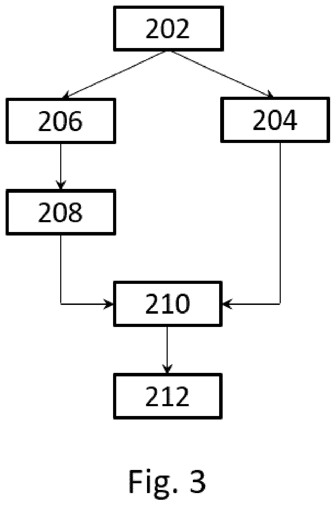

Fig. 3 shows a flowchart which illustrates a computer-implemented method of operating the medical system 100 illustrated in Fig. 1 or the medical system 300 of Fig. 2. After measuring in step 202 the B1 magnitude and B1 phase of an object in a first position, e.g., by determining them from magnetic resonance imaging data of a portion of body of the patient in head first (HF) position, in step 206 a second B1 magnitude and B1 phase measurement is performed of the object in a second position, e.g., by determining them from magnetic resonance imaging data of a portion of body of the patient in feet first (FF) position. The second position, in an example, is obtained by a 180 degrees yaw with respect to the first position, that is a 180 degrees rotation around a vertical axis for a horizontally positioned MRI system. In other words, the yaw is the rotation around an axis perpendicular to the direction of the main static magnetic field B0, e.g., around the axis perpendicular to the longitudinal axis of the bore of the MRI system.

The maps of electrical properties of both scans are (re)constructed separately, yielding artefacts from the violated TPA, with other words in step 204 the conductivity and/or permittivity maps are (re)constructed for the first position of the object, and then in step 208 the conductivity and/or permittivity maps are (re)constructed for the second position of the object.

To remove these artefacts, in step 210 the maps of electrical properties for one of the positions of the object is registered or in other words rotated back with a yaw angle of 180 degrees and then averaged with the respective maps of electrical properties for the other position of the object. In step 212 the obtained or resultant conductivity and/or the permittivity maps of the object or the corresponding data are output to a user interface, e.g., to a display screen connected to or integrated within the medical system 100 or 300.

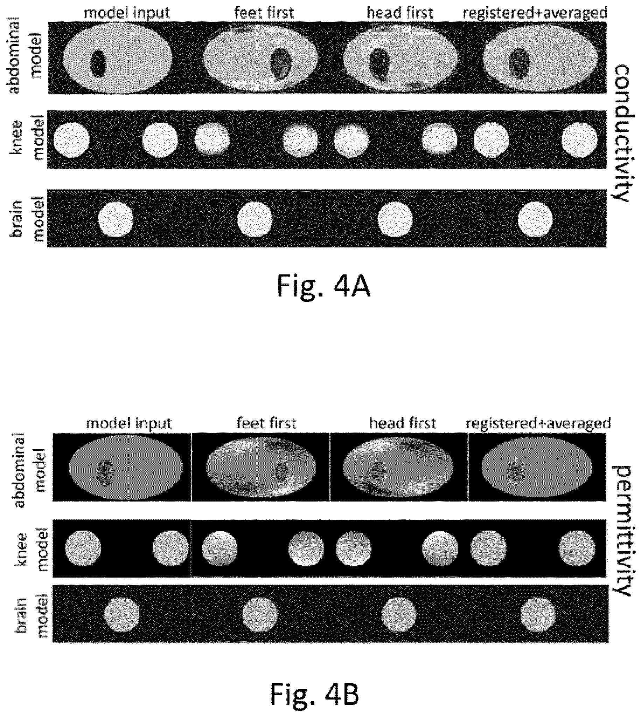

The invention is further demonstrated in Fig. 4A and Fig. 4B, by using three different application examples: abdomen, knees, and brain in a quadrature body coil (QBC) at 3 T. For each application example, simplified "in silico" models have been developed to visualize the effect, utilizing electrical properties listed in Table 1.

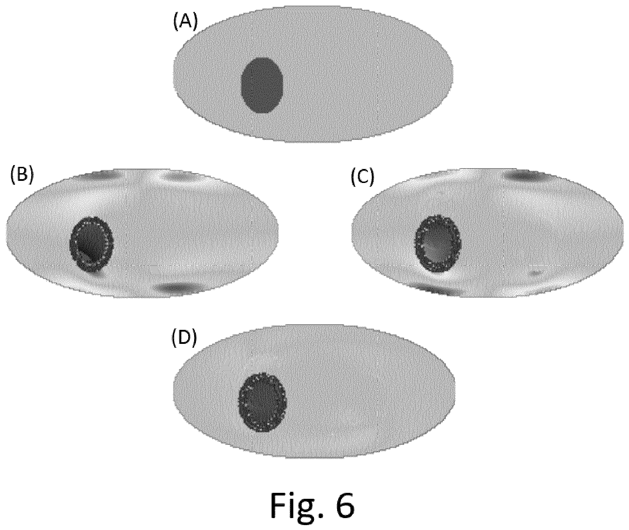

Table 1: Electrical properties used for the models shown in Figs. 4A, 4B and 6.

| Model / compartment | Conductivity [S/m] | Relative Permittivity |
|---|---|---|
| Abdomen (background) | 0.5 | 50 |
| Abdomen (inner compartment) | 0.25 | 30 |
| Knees | 0.5 | 50 |

(continued)

| Model / compartment | Conductivity [S/m] | Relative Permittivity |
|---|---|---|
| Brain | 0.5 | 50 |

To simulate (axial) MR scans, the field maps of $H^+=(H_x+iH_y)/2$ and $H^-=(H_x-iH_y)/2$ from two polarizations of the QBC have been simulated for each model and added correspondingly to obtain the transceive phase of the two patient positions HF and FF. After suitable phase unwrapping, the transceive phase has been divided by two according to the TPA to estimate the required phase $\varphi^+$ of $H^+$, which corresponds to MR measurements with, e.g., spin-echo based sequences or Steady-State Free-Precession (SSFP) sequences. The magnitude $|H^+|$ corresponds to the B1 map as measured with, e.g., Dual Refocusing Echo Acquisition Mode (DREAM) or Actual Flip angle Imaging (AFI). This magnitude, together with the estimated phase, was used as input for standard EPT reconstructions via

$$\omega\varepsilon - i\sigma = -\Delta H^+/(\mu_0\omega H^+) \qquad \text{Eq. 1}$$

with $\omega$ the Larmor frequency of the MR system applied, $\Delta$ the Laplacian operator, and $\mu_0$ the vacuum permeability. The reconstructed maps of electrical properties from the FF position were registered and averaged with the reconstructed maps of electrical properties from the HF position. Registration is performed such that underlying anatomies match, e.g., kidney voxels are averaged with kidney voxels, liver voxels with liver voxels, etc.

As demonstrated in Figs. 4A and 4B, the reconstructed maps of electrical properties from the FF position and the HF position show strong artefacts for the abdomen and knee phantom, which vanish almost completely after averaging. The brain model serves as counterexample, free of artefacts due to its spherical symmetry. These findings hold for both, conductivity and permittivity. Alternatively, instead averaging the two reconstruction results, the two measured B 1 fields may first be mapped to each other and then averaged before reconstruction, such that only a single reconstruction is required. A corresponding mapping is performed such that B 1 fields associated to underlying anatomies match.

In an alternative embodiment, for example when an open MRI scanner is utilized wherein the main static magnetic field B0 is oriented vertically, the first position is either one of supine and prone position of the patient which is thus associated with the first magnetic resonance imaging data, while the other one of supine and prone position of the patient is associated with the second magnetic resonance imaging data. The supine and prone positions are separated from each other by 180 degrees roll around the longitudinal axis of the patient. For example, when the first position is supine, the second position is prone. In this case the patient has only to turn around from lying on the back to lying on the belly, without needing to be repositioned from a HF to a FF in the scanner.

In clinical practice, an exact repositioning of the patient might be cumbersome and in fact it is not required to perform the invention. Slight imperfections due to repositioning can be compensated by a corresponding spatial translation of the maps of electrical properties after respective registering or mapping, because artefacts from TPA violation tend to be of low spatial frequencies. Therefore, the yaw angle is preferably about 180 degrees. A rotatable patient table would facilitate the workflow in order not to necessitate movements of the patients themselves and to keep their position assumed for the first measurement position with respect to the patient table, also for the second measurement position.

Although the invention is herewith demonstrated for axial slices, it is applicable for all slice orientations.

As an alternative, two MRI scanners may be used sequentially for acquiring the raw data while the B0 direction is flipped for one of the scanners, with other words having two MRI scanners with opposite directions of the main static magnetic fields. Technically, this is not very challenging and in some other applications, this might even be interesting to avoid left-right asymmetries which can occur especially at higher field strengths (i.e. at 3 T and above for body imaging, 7 T and above for head imaging). Another embodiment might allow to ramp down the magnet and then to change the direction for a second ramp. However, this may become much more time-consuming.

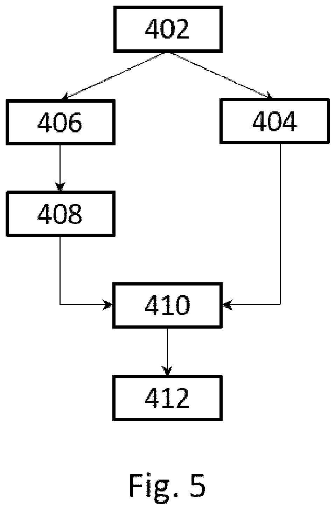

With reference to Fig. 5, a flowchart of an alternative embodiment of the invention is illustrated, showing a computer-implemented method of operating the medical system 100 illustrated in Fig. 1 or the medical system 300 of Fig. 2.

In this alternative embodiment, instead of measuring the patient twice in the respective two positions (head first and feet first), the patient is measured only once, and the second measurement is mimicked by mirroring the magnitude of the complex B1 map (i.e. the phase of the complex B 1 map remains unchanged). Reconstructing the electrical properties from the original and mirrored maps yields roughly complementary artefacts, which cancel by averaging the maps. This method reduces the scan time and releases the constraint on re-positioning the patient for the second scan, which renders this method more practical for clinical routine.

The method comprises the step 402 of measuring B 1 magnitude and B 1 phase of the object, e.g., by determining them from magnetic resonance imaging data of a portion of body of the patient. In step 404 a first conductivity map and/or a first permittivity map are (re)constructed from the measurements in step 402. Parallelly, in step 406 the B 1 magnitude measured in step 402 is mirrored and combined with the originally measured B 1 phase, from which then in step 408 a second conductivity map and/or a second permittivity map is (re)constructed. In step 410 the respective first and second maps of electrical properties are averaged to remove these artefacts, and therewith the conductivity and/or the permittivity maps of the object are obtained. In step 412 the conductivity and/or the permittivity maps of the object or the corresponding data are output to a user interface, e.g., to a display screen connected to or integrated within the medical system 100 or 300.

This alternative embodiment is demonstrated in Fig. 6 by using a simplified abdominal model in a quadrature body coil at 3 T and using the electrical properties of the inner and outer compartments of the abdomen according to Table 1. To simulate (axial) MR scans, the field maps of $H^+=(H_x+iH_y)/2$ and $H^-=(H_x-iH_y)/2$ from two polarizations of the QBC have been simulated and added correspondingly to obtain the transceive phase. After suitable phase unwrapping, the transceive phase has been divided by two according to the TPA to estimate the required phase $\varphi^+$ of $H^+$, which corresponds to MR measurements with, e.g., spin-echo based sequences or Steady-State Free-Precession (SSFP) sequences. The magnitude $|H^+|$ corresponds to the B1 map as measured with, e.g., Dual Refocusing Echo Acquisition Mode (DREAM) or Actual Flip angle Imaging (AFI). This (original) magnitude, together with the estimated phase, was used as input for the reconstruction of the first maps of electrical properties via Eq. 1.

For reconstruction of the second maps of electrical properties, the magnitude of $H^+$ was mirrored (obtaining $|H'^+|$) and combined with the original phase $\varphi^+$ (yielding the complex $H'^+$) to mimic the second position of the object. The corresponding reconstruction yields:

$$\omega\varepsilon' - i\sigma' = -\Delta H'^+/(\mu_0\omega H'^+) \qquad \text{Eq. 2}$$

The TPA-related artefacts of $\varepsilon'$ and $\sigma'$ are roughly complementary to the TPA-related artefacts of $\varepsilon$ and $\sigma$, see Fig. 6 (C) with respect to Fig. 6 (B), and thus vanish almost completely after averaging, as illustrated in Fig. 6 (D). The graphical representation in Fig. 6 (B) is derived from the original B 1 magnitude and originally measured B 1 phase and that in Fig. 6 (C) is derived from the mirrored B 1 magnitude and originally measured B 1 phase, which are both corrupted by TPA and by averaging the reconstruction results based on the original (B) and the mirrored (C) B 1 magnitudes, the resulting map of electrical property, in this case of the reconstructed conductivity map (D) of the object yields to properties close to the model input (A). This holds also for permittivity.

Alternatively, instead of averaging the two reconstruction results, the two measured B 1 fields might be averaged before reconstruction, such that only a single reconstruction is required.

In practice, $|H'^+|$ was obtained by averaging $|H^+|$ mirrored in left/right direction and anterior/posterior direction.

For non-symmetric objects, it is recommended to perform these mirroring operations line by line with individual pivot points.

Although the invention with reference to Figs. 5 and 6 has been demonstrated for axial slices, it is applicable for all slice orientations.

The invention can be applied for all kinds of electrical properties mapping, e.g., for imaging knees or legs, which show a particular violation of axial symmetry. Thus, on one hand, knees or legs suffer specific artefacts in maps of electric properties, and on the other hand, benefit particularly from the invention.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a magnetic hard disk drive, a solid state hard disk, flash memory, a USB thumb drive, Random Access Memory (RAM), Read Only Memory (ROM), an optical disk, a magneto-optical disk, and the register file of the computational system. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the reinforced multi-filar conductor bundle. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. A system (100, 300) for electrical properties tomography imaging, comprising:

    - a memory (110) storing machine executable instructions (120);
    - a computational system (104), wherein execution of the machine executable instructions causes the computational system to:

        - receive a magnetic resonance imaging data (124) of an object and ascertain a first set of B1 magnitudes and B1 phases from the magnetic resonance imaging data;
        - mirror the first set of B1 magnitudes to obtain a second set of B1 magnitudes;
        - determine a resultant map of electrical property by:

            - constructing a first map of electrical property based on the first set of B1 magnitudes and the first set of B1 phases, constructing a second map of electrical property based on the second set of B1 magnitudes and the first set of B1 phases, averaging the first and second maps of electrical property; or
            - averaging the first and second sets of B1 magnitudes, and using the first set of B1 phases;

        - output the resultant map of electrical property to a display screen.

**2.** The system of claim 1, wherein the mirroring is performed line by line with individual pivot points.

**3.** A system (100, 300) for electrical properties tomography imaging, comprising:

- a memory (110) storing machine executable instructions (120);
- a computational system (104), wherein execution of the machine executable instructions causes the computational system to:

- receive a first magnetic resonance imaging data (124) in a first position of an object and ascertain a first set of B1 magnitudes and B1 phases from the first magnetic resonance imaging data;
- receive a second magnetic resonance imaging data (124) in a second position of the object and ascertain a second set of B1 magnitudes and B1 phases from the second magnetic resonance imaging data;
- determine a resultant map of electrical property by:

- constructing first and second maps of electrical property based on the respective first set and second set of B1 magnitudes and B1 phases, registering the first map of electrical property to the second map of electrical property and averaging the registered maps of electrical property; or
- mapping the first set of B1 magnitudes and B1 phases to the second set of B1 magnitudes and B1 phases and averaging the mapped sets of B1 magnitudes and B1 phases;

- output the resultant map of electrical property to a display screen.

**4.** The system of claim 3, wherein the second position is at about 180 degrees yaw from the first position.

**5.** The system of claim 3 or claim 4, comprising a support configured for placing the object on it and which support is configured rotatable to allow orientation of the object from the first position to the second position.

**6.** The system of claim 3, comprising:

- two magnetic resonance imaging scanners placed such that the direction of the main static magnetic fields of the two scanners are opposite to each other;
- a support configured for placing the object on it, and wherein the support is movable from the first position in the first scanner to the second position in the second scanner.

**7.** The system of any of the preceding claims,

- wherein the memory is configured to store an image segmentation algorithm (122), wherein the image segmentation algorithm is configured for outputting one or more predetermined anatomical regions within the magnetic resonance imaging data (124) descriptive of a predetermined field of view (309) of the object, wherein the object is a portion of a subject (318);
- wherein execution of the machine executable instructions causes the computational system to provide an image segmentation (126) comprising the one or more anatomical regions within the magnetic resonance imaging data in response to inputting the magnetic resonance imaging data into the image segmentation algorithm;
- wherein the resultant map of electrical property is output for the provided image segmentation.

**8.** The system of any of the preceding claims, wherein the electrical property is at least one of a conductivity and a permittivity.

**9.** A method of electrical properties tomography imaging, comprising:

- receiving a magnetic resonance imaging data (124) of an object and ascertaining a first set of B1 magnitudes and B1 phases from the magnetic resonance imaging data;
- mirroring the first set of B1 magnitudes to obtain a second set of B1 magnitudes;
- determining a resultant map of electrical property by:

- constructing a first map of electrical property based on the first set of B1 magnitudes and B1 phases, constructing a second map of electrical property based on the second set of B1 magnitudes and first set of B1 phases, averaging the first and second maps of electrical property; or

- averaging the first and second sets of B1 magnitudes, and using the first set of B1 phases;

- outputting the resultant map of electrical property to a display screen.

10. The method of claim 9, wherein the mirroring is performed line by line with individual pivot points.

11. A method of electrical properties tomography imaging, comprising:

- receiving a first magnetic resonance imaging data (124) in a first position of the object and ascertaining a first set of B1 magnitudes and B1 phases from the first magnetic resonance imaging data;
- receiving a second magnetic resonance imaging data (124) in a second position of the object and ascertaining a second set of B1 magnitudes and B1 phases from the second magnetic resonance imaging data;
- determining a resultant map of electrical property by:

- constructing first and second maps of electrical property based on the respective first set and second set of B1 magnitudes and B1 phases, registering the first map of electrical property to the second map of electrical property and averaging the registered maps of electrical property; or
- mapping the first set of B1 magnitudes and B1 phases to the second set of B1 magnitudes and B1 phases and averaging the mapped sets of B1 magnitudes and B1 phases;

- outputting the resultant map of electrical property to a display screen.

12. The method of claim 11, comprising:

- rotating the object on a rotatable support between the first position and the second position with a yaw of about 180 degrees; or
- providing the first and the second magnetic resonance data with two respective magnetic resonance imaging scanners placed such that the direction of the main static magnetic fields of the two scanners are opposite to each other; and moving the object on a support from the first position in the first scanner to the second position in the second scanner.

13. The method of any of the claims 9 to 12, comprising:

- providing an image segmentation (126) comprising one or more anatomical regions within the magnetic resonance imaging data in response to inputting the magnetic resonance imaging data into an image segmentation algorithm;
- wherein outputting the resultant map of electrical property is for the provided image segmentation.

14. The method of any of the claims 9 to 13, wherein the electrical property is at least one of a conductivity and a permittivity.

15. A computer program comprising machine executable instructions, wherein execution of the machine executable instructions causes a computational system to perform the steps of a method according to any of the claims 9 to 14.

Fig. 1

Fig. 2

Fig. 3

Fig. 4A

Fig. 4B

402

406          404

408

410

412

Fig. 5

(A)

(B)　　　　　　　　　　　　　　(C)

(D)

# Fig. 6

Europäisches
Patentamt

European
Patent Office

Office européen
des brevets

# EUROPEAN SEARCH REPORT

Application Number

**EP 23 21 0349**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/308148 A1 (KATSCHER ULRICH [DE] ET AL) 29 September 2022 (2022-09-29) * paragraph [0012] – paragraph [0024] * ----- | 1-15 | INV. G01R33/24 A61B5/0536 A61B5/055 |
| A | LIU JIAEN ET AL: "In vivo imaging of electrical properties of an animal tumor model with an 8-channel transceiver array at 7?T using electrical properties tomography", MAGNETIC RESONANCE IN MEDICINE, vol. 78, no. 6, 23 January 2017 (2017-01-23), pages 2157-2169, XP093151173, US ISSN: 0740-3194, DOI: 10.1002/mrm.26609 Retrieved from the Internet: URL:https://onlinelibrary.wiley.com/doi/full-xml/10.1002/mrm.26609> * page 2157 – page 2162; figure 1 * ----- | 1-15 | G01R33/48 |

|  | TECHNICAL FIELDS SEARCHED (IPC) |
|---|---|
|  | G01R A61B |

The present search report has been drawn up for all claims

| Place of search | Date of completion of the search | Examiner |
|---|---|---|
| Munich | 15 April 2024 | Lebar, Andrija |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ..................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 0349

This annex lists the patent family members relating to the patent documents cited in  the above-mentioned European search report.
The members are as contained in the European Patent Office EDP file on
The European Patent Office is in no way liable for these particulars which are merely given for the purpose of information.

15-04-2024

| Patent document cited in search report | Publication date | Patent family member(s) | Publication date |
|---|---|---|---|
| US 2022308148 A1 | 29-09-2022 | EP 3754357 A1 | 23-12-2020 |
| | | US 2022308148 A1 | 29-09-2022 |
| | | WO 2020254571 A1 | 24-12-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEIJSEN R. et al.** Electrical Properties Tomography: A Methodological Review. *Diagnostics*, 2021, vol. 11, 176, https://doi.org/10.3390/diagnostics11020176